# EUROPÄISCHE PATENTSCHRIFT

(11) **EP 0 648 756 B1**
(45) Veröffentlichungstag und Bekanntmachung des Hinweises auf die Patenterteilung: **24.03.1999**
(21) Anmeldenummer: 94113935.4
(22) Anmeldetag: 06.09.1994
(51) Int. Cl.: C07D 251/70, C08K 5/34

(54) **Verfahren zur Herstellung von Hydroxyoxaalkylmelaminen**
Process for the production of Hydroxyoxaalkylmelamines
Procédé de préparation de hydroxyalkyl mélamines

(30) Priorität: 15.09.1993 DE 4331233
(43) Veröffentlichungstag der Anmeldung: 19.04.1995
(73) Patentinhaber: BASF Aktiengesellschaft, 67063 Ludwigshafen (DE)
(72) Erfinder: Guenther, Erhard Dr., D-67063 Ludwigshafen (DE); Reuther, Wolfgang, D-69118 Heidelberg (DE); Scherr, Günter, D-67065 Ludwigshafen (DE); Dimmler, Manfred Dr., D-67125 Dannstadt-Schauerheim (DE)

(56) Entgegenhaltungen:
- EP-A- 0 051 753
- EP-A- 0 166 297
- EP-A- 0 178 611
- EP-A- 0 179 408
- EP-A- 0 225 433
- EP-A- 0 408 947

## Beschreibung

Die Erfindung betrifft ein verbessertes Verfahren zur Herstellung von Hydroxyoxaalkylmelaminen.

Hydroxyoxaalkylmelamine sind wertvolle Zwischenprodukte zur Herstellung von Urethanen und eignen sich außerdem hervorragend zur Modifizierung von Aminoplastharzen (vgl. EP-A-408 947).

Aus der EP-A-225 433 ist ein Verfahren zur Herstellung von Hydroxyoxaalkylmelaminen der allgemeinen Formel I 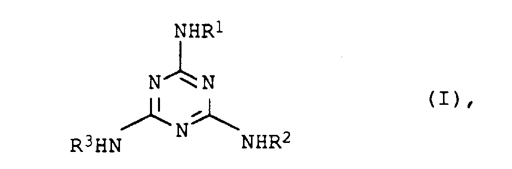 worin R¹ einen Rest der allgemeinen Formel II bedeutet

H(̵O-CHR'-CHR')̵ₙ (II),

in der die Reste R' gleich oder verschieden sein können und für Wasserstoff oder C₁-C₄-Alkyl stehen und n die Bedeutungen 2 oder 3 besitzt und die Reste R² und R³ für einen der Reste R¹ oder Wasserstoff stehen, durch Umsetzung von Melamin mit einer Verbindung der allgemeinen Formel III,

H(̵O-CHR'-CHR')̵ₙ-NH₂ (III),

worin R' und n die oben angegebenen Bedeutungen besitzen, bei einer Temperatur von 120 bis 250°C in Gegenwart eines sauren Katalysators bekannt.

Der saure Katalysator wird hierbei in einer Menge von 0,05 bis 3 mol, vorzugsweise 0,1 bis 1 mol, bezogen auf 1 mol Melamin eingesetzt. Mit zunehmender Katalysatormenge ist ein Anstieg der Reaktionsgeschwindigkeit zu beobachten.

Als saure Katalysatoren kommen gemäß der EP-A-225 433 alle starken und mittelstarken Protonsäuren in Betracht, z.B. Flußsäure, Salzsäure, Bromwasserstoffsäure, Schwefelsäure, Salpetersäure, Phosphorsäure, Amidosulfonsäure, Thiocyansäure, p-Toluolsulfonsäure oder Methansulfonsäure sowie Lewis-Säuren, wie Bortrifluorid, Aluminiumchlorid, Zinn(IV)chlorid, Antimon(V)fluorid oder Eisen(III)bromid.

Aufgabe der vorliegenden Erfindung war es nun, dieses bekannte Verfahren in Hinblick auf eine Verringerung der insgesamt zur Produktion erforderlichen Zeit weiter zu verbessern. Hierbei sollte sich gleichzeitig eine verbesserte Produktqualität ergeben.

Diese Aufgabe wurde überraschend gelöst durch ein Verfahren zur Herstellung von Hydroxyoxaalkylmelaminen der allgemeinen Formel I 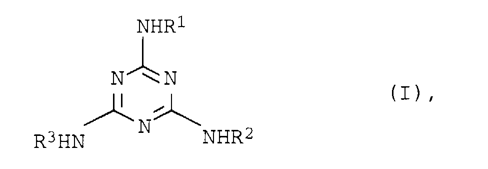 worin R¹ einen Rest der allgemeinen Formel II bedeutet

H(̵O-CHR'-CHR')̵ₙ (II),

in der die Reste R' gleich oder verschieden sein können und für Wasserstoff oder C₁-C₄-Alkyl stehen und n die Bedeutungen 2 oder 3 besitzt und die Reste R² und R³ für einen der Reste R¹ oder Wasserstoff stehen, durch Umsetzung von Melamin mit einer Verbindung der allgemeinen Formel III,

H(̵O-CHR'-CHR')̵ₙ-NH₂ (III),

worin R' und n die oben angegebenen Bedeutungen besitzen, bei einer Temperatur von 120 bis 250°C in Gegenwart eines sauren Katalysators, das dadurch gekennzeichnet ist, daß man als sauren Katalysator Hypophosphorige Säure (H₃PO₂) in einer Menge von 0,01 bis 0,15, vorzugsweise 0,02 bis 0,12 und besonders bevorzugt 0,03 bis 0,1 mol, bezogen auf 1 mol Melamin, einsetzt.

Als sauren Katalysator kann man Hypophosphorige Säure allein oder zusammen mit einer starken oder mittelstarken Protonsäure oder Lewissäure, vorzugsweise einer starken oder mittelstarken Protonsäure, wie Phosphorsäure oder Ammoniumchlorid, einsetzen.

Bevorzugt werden mit dem erfindungsgemäßen Verfahren Hydroxyoxaalkylmelamine der allgemeinen Formel I hergestellt, bei denen R' für Wasserstoff steht und n die Bedeutung 2 besitzt.

Dies sind die Verbindungen N-Mono-(5-hydroxy-3-oxapentyl)melamin, N,N'-Bis-(5-hydroxy-3-oxapentyl)melamin, N,N',N"-Tris-(5-hydroxy-3-oxapentyl)melamin, sowie deren Mischungen.

Die als Ausgangsverbindungen eingesetzten Oxaalkanolamine der allgemeinen Formel III sind entweder bekannt oder lassen sich nach bekannten Methoden herstellen, vgl. M.S. Malinskii, A.N. Korchagina, A.G. Yudasina, D.G. Yurko Vorpr. Khim. Khim. Teknol. 1974, 34, 6-11 (Russ.) und JP-A-79-3005.

Das Verfahren wird im allgemeinen so durchgeführt, daß man ein Gemisch aus Melamin, Oxaalkanolamin (beispielsweise 2,2'-Aminoethoxyethanol), saurem Katalysator und gegebenenfalls einem Lösungsmittel vorlegt und unter Rühren auf eine Temperatur von 120 bis 250°C, insbesondere von 150 bis 230°C erhitzt.

Man arbeitet im allgemeinen bei atmosphärischen Druck. Für den oberen Temperaturbereich (230 bis 250°C) muß jedoch in der Regel ein Druck von 1 bis 15 bar eingehalten werden.

Weiterhin empfiehlt es sich, die Umsetzung in Gegenwart eines Schutzgases durchzuführen. Das Schutzgas wird dabei im allgemeinen über die Oberfläche der Reaktionsmischung geleitet. Geeignete Schutzgase sind z.B. Edelgase und insbesondere Stickstoff.

Das erfindungsgemäße Verfahren wird vorzugsweise in Abwesenheit von Lösungsmitteln durchgeführt, jedoch ist es auch möglich, in einem organischen Lösungsmittel zu arbeiten. Hierfür sind insbesondere Polyole, z.B. Ethylenglykol, 1,2-Propylenglykol, Diethylenglykol oder Triethylenglykol geeignet.

Die verwendete Menge an Oxaalkanolamin (z.B. 2,2'-Aminoethoxyethanol) kann beliebig gewählt werden. Vorzugsweise wird jedoch mit einem Aminüberschuß gearbeitet. Es werden gewöhnlich 3 bis 10 mol Amin pro Mol Melamin eingesetzt.

Der Reaktionsverlauf kann mit analytischen Methoden, beispielsweise mittels Hochdruckflüssigkeitschromatographie (HPLC) verfolgt werden. Man kann die Reaktion bei jedem beliebigen Umsatzgrad abbrechen, wobei man beispielsweise Gemische aus N,N',N"-Tris-(5-hydroxy-3-oxapentyl)melamin, N,N'-Bis-(5-hydroxy-3-oxapentyl)melamin und gegebenenfalls auch N-Mono-(5-hydroxy-3-oxapentyl)melamin erhält, die eine definierte, reproduzierbare Zusammensetzung aufweisen.

Bei vollständigem Umsatz erhält man hierbei reines N,N',N"-Tris-(5-hydroxy-3-oxapentyl)melamin.

Zur Isolierung der Wertprodukte neutralisiert man zweckmäßig die jeweilige Katalysatorsäure, indem man eine Base, z.B. Natriumhydroxid, Kaliumhydroxid, Natriumcarbonat, Kaliumcarbonat, Calciumcarbonat oder Bariumcarbonat zur Reaktionsmischung gibt.

Der Vorteil des erfindungsgemäßen Verfahrens besteht nun zusätzlich darin, daß auf die Abtrennung der hierbei anfallenden Salze durch Filtration verzichtet werden kann.

Für die großtechnische Produktion bedeutet die Filtration einen erheblichen Verfahrensaufwand, da beispielsweise die bei der Neutralisation anfallenden Salze in Aminoethoxyethanol als Solvens sehr feinkristallin anfallen und lange Filtrationszeiten und damit Produktionszeiten bedingen.

Wenn der saure Katalysator als Ammoniumverbindung eingesetzt wird, muß darüber hinaus die Filtration wegen der durch gelösten Ammoniak bedingten Geruchsbelästigung in einem gekapselten Filteraggregat erfolgen, oder das Ammoniak durch Anlegen von Vakuum vor der Filtration entfernt werden, was ebenfalls längere Produktionszeiten zur Folge hat.

Um eine ausreichend niedrige Viskosität der Filtrationslösung zu erzielen, muß das Oxaalkanolamin außerdem in großem Überschuß zu Melamin eingesetzt werden. Dies bedingt eine relativ niedrige Kapazität pro Kesselvolumen.

Im erfindungsgemäßen Verfahren kann daher bei einem deutlich vergrößerten Anteil an Melamin verglichen mit Oxaalkanolamin (beispielsweise 3 bis 5 mol Oxaalkanolamin auf 1 mol Melamin) gearbeitet werden. Dies führt zu einer signifikanten Steigerung der Kapazität bei gleichem Kesselvolumen.

Nach der Neutralisation kann das überschüssige Oxaalkanolamin beispielsweise bei vermindertem Druck (ca. 10 mbar) bei einer Temperatur von ca. 190°C abdestilliert werden, wobei der annähernd farblose Rückstand zu einem Harz erstarrt.

Hierbei wurde überraschend gefunden, daß die nach dem erfindungsgemäßen Verfahren hergestellten Hydroxyoxaalkylmelamine deutlich heller sind (Farbzahl 0 bis 1) als bei Herstellung nach herkömmlichen Verfahren (Farbzahl 80 bis 150).

Das erfindungsgemäße Verfahren erlaubt darüber hinaus eine leichtere Einstellung einer bestimmten Produktzusammensetzung. Wegen des Wegfalls der Filtration verringert sich insbesondere die insgesamt zur Produktion erforderliche Zeit um ca. 25 %.

Die Prozentangaben in den Beispielen beziehen sich auf das Gewicht.

### Beispiele

### Beispiel 1

Zu einer Mischung von 630 g (5,00 mol) Melamin und 1575 g (15 mol) 2,2'-Aminoethoxyethanol wurden unter Rühren 5,0 g (30 mmol) Hypophosphorige Säure (50 % in Wasser) sowie 9,25 g (173 mmol) Ammoniumchlorid gegeben. Dann wurde unter Überleiten eines schwachen Stickstoffstromes bis zur gewünschten Zusammensetzung (Mono:Bis:Tris-5-hydroxy-3-oxapentyl-melamin = 10:50:40 mol-%, HPLC-Kontrolle) bei 195°C gerührt. Anschließend wurde das überschüssige Amin im Vakuum abdestilliert. Nach Abkühlen auf 90°C wurde mit 16 g Natronlauge (50 %ig) neutralisiert. Es wurden 2405 g eines farblosen Harzes erhalten (Farbzahl: 0 bis 1; pH = 10,8).

### Beispiel 2

Zu einer Mischung von 630 g (5,00 mol) Melamin und 1575 g (15 mol) 2,2'-Aminoethoxyethanol wurden unter Rühren 5,0 g (30 mmol) Hypophosphorige Säure (50 % in Wasser) sowie 20 g (173 mmol) Phosphorsäure (85 % in Wasser) gegeben.

## Patentansprüche

1. Verfahren zur Herstellung von Hydroxyoxaalkylmelaminen der allgemeinen Formel I 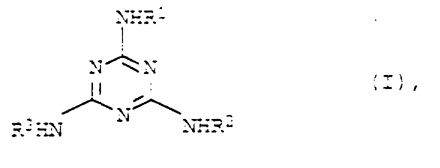 worin R¹ einen Rest der allgemeinen Formel II bedeutet
H(̵O-CHR'-CHR')̵ₙ (II),
in der die Reste R' gleich oder verschieden sein können und für Wasserstoff oder C₁-C₄-Alkyl stehen und n die Bedeutungen 2 oder 3 besitzt und die Reste R² und R³ für einen der Reste R¹ oder Wasserstoff stehen, durch Umsetzung von Melamin mit einer Verbindung der allgemeinen Formel III,
H(̵O-CHR'-CHR')̵ₙ-NH₂ (III),
worin R' und n die oben angegebenen Bedeutungen besitzen, bei einer Temperatur von 120 bis 250°C in Gegenwart eines sauren Katalysators, dadurch gekennzeichnet, daß man als sauren Katalysator Hypophosphorige Säure (H₃PO₂) in einer Menge von 0,01 bis 0,15 mol, bezogen auf 1 mol Melamin einsetzt.

2. Verfahren nach Anspruch 1, dadurch gekennzeichnet, daß man als sauren Katalysator Hypophosphorige Säure in einer Menge von 0,01 bis 0,15 mol, bezogen auf 1 mol Melamin, zusammen mit einer starken oder mittelstarken Proton säure oder Lewissäure einsetzt.

3. Verfahren nach Anspruch 2, dadurch gekennzeichnet, daß man als starke oder mittelstarke Protonsäure Phosphorsäure oder Ammoniumchlorid einsetzt.

4. Verfahren nach den Ansprüchen 1 bis 3, dadurch gekennzeichnet, daß R' für Wasserstoff steht, und n die Bedeutung zwei (2) besitzt.

## Claims

1. A process for preparing hydroxyoxaalkylmelamines of the formula I 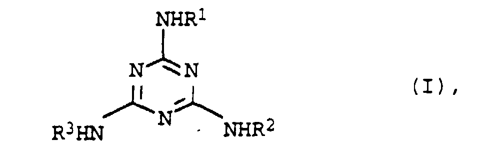 where R¹ is a radical of the formula II
H(̵O-CHR'-CHR')̵ₙ (II),
where the radicals R' can be identical or different and are hydrogen or C₁-C₄-alkyl and n is 2 or 3, and the radicals R² and R³ are one of the radicals R¹ or hydrogen, by reacting melamine with a compound of the formula III
H(̵O-CHR'-CHR')̵ₙ-NH₂ (III),
where R' and n are as defined above, at from 120 to 250°C in the presence of an acid catalyst, wherein hypophosphorous acid (H₃PO₂) in an amount of from 0.01 to 0.15 mol, based on one mol of melamine, is used as acid catalyst.

2. A process as claimed in claim 1, wherein hypophosphorous acid in an amount of from 0.01 to 0.15 mol, based on 1 mol of melamine, together with a strong or intermediate-strength protic acid or Lewis acid are used as acid catalyst.

3. A process as claimed in claim 2, wherein phosphoric acid or ammonium chloride is used as strong or intermediate-strength protic acid.

4. A process as claimed in any of claims 1 to 3, wherein R' is hydrogen and n is two (2).

## Revendications

1. Procédé de préparation d'hydroxyoxaalkylmélamines de la formule générale I : 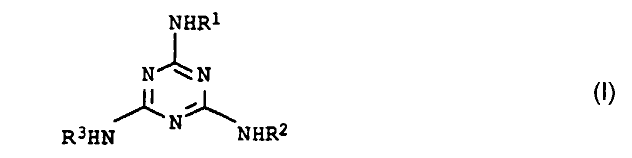 dans laquelle R¹ représente un radical de la formule générale II :
H(̵O-CHR'-CHR')̵ₙ (II)
dans laquelle les radicaux R' peuvent être identiques ou différents et représentent de l'hydrogène ou un alkyle en C₁-C₄, et n a les significations de 2 ou de 3 et les radicaux R² et R³ représentent un des radicaux R¹ ou de l'hydrogène, par réaction de mélamine avec un composé de la formule générale III :
H(̵O-CHR'-CHR')̵ₙ-NH₂ (III)
dans laquelle R' et n ont les significations indiquées ci-dessus, à une température de 120 à 250°C en présence d'un catalyseur acide, caractérisé en ce qu'on met en oeuvre, comme catalyseur acide, de l'acide hypophosphoreux (H₃PO₂) en une quantité de 0,01 à 0,15 mole, par rapport à 1 mole de mélamine.

2. Procédé suivant la revendication 1, caractérisé en ce que, comme catalyseur acide, on met en oeuvre de l'acide hypophosphoreux en une quantité de 0,01 à 0,15 mole, par rapport à 1 mole de mélamine, conjointement à un acide de Lewis ou acide protonique fort ou moyennement fort.

3. Procédé suivant la revendication 2, caractérisé en ce que, comme acide protonique fort ou moyennement fort, on met en oeuvre de l'acide phosphorique ou du chlorure d'ammonium.

4. Procédé suivant l'une des revendications 1 à 3, caractérisé en ce que R' représente de l'hydrogène, et n a la signification de deux (2).
